# EUROPÄISCHE PATENTSCHRIFT

(11) **EP 0 483 197 B1**
(45) Veröffentlichungstag und Bekanntmachung des Hinweises auf die Patenterteilung: **25.01.1995**
(21) Anmeldenummer: 90910713.8
(22) Anmeldetag: 10.07.1990
(51) Int. Cl.: C07C 59/125, C07C 59/90, C07C 69/18

(54) **ALKOXILIERUNGSPRODUKTE VON OH-GRUPPENHALTIGEN CARBONSÄUREDERIVATEN UND/ODER CARBONSÄUREN**
ALKOXYLATION PRODUCTS OF CARBOXYLIC ACIDS AND/OR CARBOXYLIC ACID DERIVATIVES CONTAINING OH GROUPS
PRODUITS D'ALKOXYLATION DE DERIVES D'ACIDES CARBOXYLIQUES ET/OU D'ACIDES CARBOXYLIQUES CONTENANT DES GROUPES OH

(30) Priorität: 14.07.1989 DE 3923394
(43) Veröffentlichungstag der Anmeldung: 06.05.1992
(73) Patentinhaber: Henkel Kommanditgesellschaft auf Aktien, 40191 Düsseldorf (DE)
(72) Erfinder: DAUTE, Peter, D-4300 Essen 1 (DE); STOLL, Gerhard, D-4052 Korschenbroich 1 (DE)
(86) Internationale Anmeldenummer: EP9001121
(87) Internationale Veröffentlichungsnummer: WO9101291

(56) Entgegenhaltungen:
- EP-A- 0 022 236
- EP-A- 0 127 810
- EP-A- 0 280 145
- DE-A- 2 911 241

## Beschreibung

Die Erfindung betrifft Alkoxylierungsprodukte von C₁₀₋₂₂-Carbonsäuremono-, -di- und/oder -triglyceriden natürlichen Ursprungs sowie ein Verfahren zur Herstellung dieser Alkoxylierungsprodukte.

Ethoxylierte und/oder propoxylierte Ricinusöle werden beispielsweise in kosmetischen Zubereitungen, Waschmitteln oder Schmierölen oder als Antistatica für Nylonteppiche eingesetzt (Kirk-Othmer: "Encyclopedia of Chemical Technology", Band 5, Seite 9, John Wiley New York (1979)). Bei Verwendung ethoxylierter und/oder propoxylierter Rizinusöle muß jedoch in Kauf genommen werden, daß die auf dem Markt erhältlichen Mengen an Rizinusöl und damit auch an ethoxylierten und/oder propoxylierten Rizinusölen starken Schwankungen unterworfen sind. Mißernten in den Hauptanbaugebieten Brasilien und Indien führen in mehr oder weniger großen Abständen zu einer Verknappung des Ausgangsmaterials Rizinusöl. Es besteht daher ein Bedürfnis nach einem gleichwertigen Ersatz für alkoxylierte Rizinusöle. Vor allem sollte das Austauschprodukt von einer breiteren, weniger krisenanfälligen Rohstoffbasis aus zugänglich sowie ökologisch und toxikologisch unbedenklich sein.

Aus DE-A-29 11 241 sind ethoxylierte Monohydroxyfettsäuren, ein Verfahren zu deren Herstellung und ihre Anwendung als Lösungsvermittler bekannt.

Es wurde nun gefunden, daß bestimmte OH-gruppenhaltige Carbonsäuren und/oder OH-gruppenhaltige Carbonsäurederivate, die mit Alkylenoxiden alkoxyliert sind, als Ersatz für alkoxylierte Rizinusöle eingesetzt werden können.

Erfindungsgegenstand sind dementsprechend Alkoxylierungsprodukte, erhältlich durch Umsetzung von Ethylenoxid, Propylenoxid und/oder Butylenoxid mit C₁₀₋₂₂-Carbonsäuremono-, -di- und/oder -triglyceriden natürlichen Ursprungs, die in Stellung 9, 10 und/oder 13, 14 Struktureinheiten der allgemeinen Formel 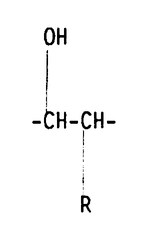
enthalten, in der
- R: ein Wasserstoffatom, eine OH-Gruppe oder eine OR¹-Gruppe
- R¹: eine Alkylgruppe mit 9 bis 18 C-Atomen, eine Alkenylgruppe mit 9 bis 18 C-Atomen oder eine 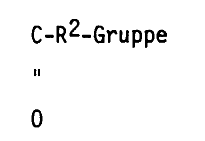 und
- R²: ein Wasserstoffatom, eine Alkylgruppe mit 1 bis 21 C-Atomen oder eine Alkenylgruppe mit 2 bis 21 C-Atomen

bedeuten.

Weiterer Erfindungsgegenstand ist ein Verfahren zur Herstellung von Alkoxylierungsprodukten, welches dadurch gekennzeichnet ist, daß C₁₀₋₂₂-Carbonsäuremono-, -di- und/oder -triglyceride, die Carbonsäurereste enthalten, die in Stellung 9, 10 und/oder 13, 14 Struktureinheiten der allgemeinen Formel 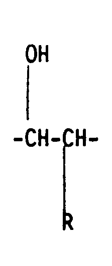
enthalten, in der
- R: ein Wasserstoffatom, eine OH-Gruppe oder eine OR¹-Gruppe,
- R¹: eine Alkylgruppe mit 9 bis 18 C-Atomen, eine Alkenylgruppe mit 9 bis 18 C-Atomen oder eine 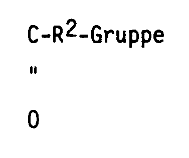 und
- R²: ein Wasserstoffatom, eine Alkylgruppe mit 1 bis 21 C-Atomen oder eine Alkenylgruppe mit 2 bis 21 C-Atomen

bedeuten, mit Ethylenoxid, Propylenoxid und/oder Butylenoxid bei Temperaturen zwischen 110 und 200 °C und Drücken zwischen 10⁵ und 2 · 10⁶ Pa umgesetzt werden.

Die erfindungsgemäßen Alkoxylierungsprodukte können nach gängigen organischen Synthesemethoden hergestellt werden. Als Ausgangsmaterialien für die Herstellung der erfindungsgemäßen Verbindungen eignen sich alle C₁₀₋₂₂-Carbonsäuremono-, -di- und/oder -triglyceride natürlichen Ursprungs mit wenigstens einer oder zwei Doppelbindungen in 9- und/oder 13-Stellung. Als Mono-, Di- und/oder Triglyceride, die OH-gruppenfreie, ungesättigte C₁₀₋₂₂-Carbonsäurereste mit wenigstens einer oder zwei Doppelbindungen in 9- und/oder 13-Stellung enthalten, eignen sich insbesondere Fette und/oder Öle natürlichen Ursprungs, deren Carbonsäuregehalt sich überwiegend aus ungesättigten C₁₀₋₂₂-Carbonsäuren mit wenigstens einer oder zwei Doppelbindungen in 9- und/oder 13-Stellung, vorzugsweise überwiegend aus ungesättigten C₁₆₋₂₂-Carbonsäuren mit wenigstens einer oder zwei Doppelbindungen in 9- und/oder 13-Stellung zusammensetzt, wie Olivenöl, Leinöl, Sonnenblumenöl, Safloröl, Sojaöl, Erdnußöl, Baumwollsaatöl, erucasäurereiches und/oder erucasäurearmes Rüböl, Palmöl, Schmalz und/oder Talg.

Die Epoxidierung der Doppelbindungen der erfindungsgemäß eingesetzten Edukte kann beispielsweise nach dem in DE-PS 857 364 beschriebenen Verfahren durch Umsetzung mit Peressigsäure in Anwesenheit saurer Katalysatoren oder mit in situ aus Ameisensäure und Wasserstoffperoxid gebildeter Perameisensäure erfolgen. Die Jodzahlen der erhaltenen Epoxidierungsprodukte liegen unterhalb 20, vorzugsweise unterhalb 15.

Anschließend werden die Oxiranringe der epoxidierten Carbonsäurederivate und/oder Carbonsäuren durch Umsetzung mit Wasserstoff oder protischen Verbindungen, wie Wasser, gerad- und/oder verzweigtkettigen Alkyl- und/oder -Alkenylalkoholen oder gerad- und/oder verzweigtkettigen, gesättigten und/oder ungesättigten C₁₋₂₂-Carbonsäuren, unter Ausbildung von Hydroxygruppen aufgespalten. Die Bedingungen der Aufspaltung werden so gewählt, daß die vorhandenen Säurederivat- und Säuregruppierungen intakt bleiben.

Die Hydrierung von epoxidierten Vorstufen kann beispielsweise analog dem in DE-OS 20 21 530 beschriebenen Verfahren in Gegenwart von Katalysatoren auf Basis von Schwermetallen der VIII. Gruppe des Periodensystems bei Temperaturen zwischen 100 und 250 °C unter Wasserstoffdrücken zwischen 10⁶ und 5 · 10⁶ Pa durchgeführt werden.

Die Umsetzungen der epoxidierten Vorstufen mit protischen Verbindungen kann gemäß den in M. S. Malinovskii "Epoxides and their Derivatives", Sivon Press 1965 beschriebenen Verfahren bei Temperaturen zwischen 50 und 200 °C und Drücken zwischen 10⁵ und 10⁶ Pa durchgeführt werden. Ringöffnungen mit gerad- und/oder verzweigtkettigen Alkyl- und/oder Alkenylalkoholen werden vorzugsweise in Gegenwart saurer Katalysatoren, wie Schwefelsäure und/oder p-Toluolsulfonsäure durchgeführt. Alkyl- und/oder Alkenylalkohole mit 9 bis 18 C-Atomen werden zur Spaltung der Oxiranringe epoxidierter Mono-, Di- und/oder Trygliceride eingesetzt. Die Oxiranringe der übrigen Vorstufen können mit Alkylalkoholen mit 1 bis 18 C-Atomen und/oder Alkenyalkoholen mit 2 bis 18 C-Atomen aufgespalten werden.

Die durch Aufspaltung der Oxiranringe erhältlichen C₁₀₋₂₂-Carbonsäuremono-, -di- und/oder -triglyceride, die Carbonsäurereste mit wenigstens einer OH-Gruppe in 9-, 10-, 13- und/oder 14-Stellung enthalten, werden anschließend nach bekannten großtechnischen Verfahren mit Ethylenoxid, Propylenoxid und/oder Butylenoxid, vorzugsweise mit Ethylenoxid und/oder Propylenoxid, vorzugsweise in Gegenwart von Katalysatoren, beispielsweise Kaliumhydroxid und/oder Natriummethylat, bei Temperaturen zwischen 110 und 200 °C, vorzugsweise zwischen 140 und 175 °C und bei Drücken zwischen 10⁵ und 2 · 10⁶ Pa, vorzugsweise zwischen 3 · 10⁵ und 5 · 10⁵ Pa alkoxyliert (siehe beispielsweise in "Chemische Technologie", Band 7, Seiten 131 bis 132, Carl-Hanser-Verlag, München/Wien (1986)).

Der Alkylenoxidgehalt der erfindungsgemäßen Verbindungen liegt zwischen 2 und 400 Gew.-%, vorzugsweise zwischen 40 und 200 Gew.-%, jeweils bezogen auf die nichtalkoxylierten Verbindungen.

### Beispiele

### 1. Herstellung von ethoxyliertem, OH-gruppenhaltigem Sojaöl aus hydriertem Sojaölepoxid

In einem Autoklaven wurden 20 kg epoxidiertes Sojaöl (ungefähre Fettsäurezusammensetzung: 8 Gew.-% Palmitinsäure, 4 Gew.-% Stearinsäure, 28 Gew.-% Ölsäure, 53 Gew.-% Linolsäure und 6 Gew.-% Linolensäure; Epoxidgehalt = 6,78 Gew.-%; Jodzahl= 5; Säurezahl= 0,4) und 0,2 kg eines Nickelkatalysators (Trägermaterial: Kieselgur) vorgelegt, die im Reaktor vorhandene Luft durch Stickstoffspülung verdrängt und bei 150 bis 170 °C mit einem Wasserstoffdruck von 2 . 10⁶ Pa bis zum Ende der Wasserstoffaufnahme (ca. 6 Stunden) hydriert. Nach Abkühlen und Abtrennen des Katalysators wurden 20 kg farbloses hydriertes Sojaölepoxid mit einer OH-Zahl (OHZ) von 165,8, einer Verseifungszahl (VZ) von 181,4, einer Jodzahl (JZ) von 8,3 und einer Säurezahl (SZ) von 1 erhalten.

650 g des hydrierten Sojaölepoxids wurden mit 5,0 g einer 30 gew.-%igen Lösung von Kaliumhydroxid in Methanol versetzt und in einem Autoklaven auf 100 °C erhitzt. Bei dieser Temperatur wurden die vorhandenen Methanolspuren durch fünfmaliges Evakuieren und Belüften mit Stickstoff entfernt. Nach Erhöhung der Reaktionstemperatur auf 150 °C wurden insgesamt 308 g Ethylenoxid portionsweise zudosiert, so daß der Druck im Reaktor einen Wert von 5 · 10⁵ Pa nicht überstieg. Nach Beendigung der Reaktion wurde auf etwa 90 °C abgekühlt und zur Abtrennung noch vorhandener Ethylenoxidspuren ca. 15 Minuten evakuiert. Es wurde eine klare gelbe Flüssigkeit mit einer OHZ von 124,5 erhalten.

### 2. Herstellung von ethoxyliertem und propoxyliertem, OH-gruppenhaltigem Sojaöl aus hydriertem Sojaölepoxid

371 g des gemäß Beispiel 1 hydrierten Sojaölepoxids wurden mit 6,2 g einer 30 gew.-%igen Lösung von Kaliumhydroxid in Methanol versetzt und unter den in Beispiel 1 angegebenen Bedingungen zuerst mit 440 g Ethylenoxid und anschließend im gleichen Reaktor mit 232 g Propylenoxid umgesetzt. Nach Entfernung der Propylenoxidspuren im Vakuum und Neutralisation des Katalysators mit 3,3 g Milchsäure wurde eine goldgelbe Flüssigkeit mit einer OHZ von 72,1 erhalten.

### 3. Herstellung von ethoxyliertem OH-gruppenhaltigem Leinöl aus hydriertem Leinölepoxid

Gemäß Beispiel 1 wurden 1 200 g epoxidiertes Leinöl (ungefähre Fettsäurezusammensetzung: 5 Gew.-% Palmitinsäure, 4 Gew.-% Stearinsäure, 22 Gew.-% Ölsäure, 17 Gew.-% Linolsäure und 52 Gew.-% Linolensäure; Epoxidgehalt: 8,9 Gew.-%; Jodzahl= 10; Säurezahl= 0,7) und 15 g eines Nickelkatalysators (Trägermaterial: Kieselgur) in einem Autoklaven vorgelegt, die im Reaktor vorhandene Luft durch Stickstoffspülung verdrängt und bei 150 bis 170 °C mit einem Wasserstoffdruck von 2 · 10⁶ Pa bis zum Ende der Wasserstoffaufnahme hydriert. Nach Abkühlen und Abtrennen des Katalysators wurde farbloses hydriertes Leinölepoxid mit einer OHZ von 202,6, einer VZ von 178,2, einer JZ von 16,9 und einer SZ von 0,7 erhalten.

650 g des hydrierten Leinölepoxids wurden gemäß Beispiel 1 mit 308 g Ethylenoxid umgesetzt. Es wurde eine gelbe Flüssigkeit mit einer OHZ von 152 erhalten.

### 4. Herstellung von ethoxyliertem OH-gruppenhaltigem Sojaöl aus mit Carbonsäuren umgesetztem Sojaölepoxid

In einem Rührkessel wurden 126 kg (805 Mol) einer Mischung gesättigter Fettsäuren (60 Gew.-% Octansäure, 35 Gew.-% Decansäure, 3 Gew.-% Dodecansäure und 2 Gew.-% Hexansäure; SZ = 361,9, JZ <1) und 180 kg (766 Mol) epoxidiertes Sojaöl (Kenndaten wie in Beispiel 1 angegeben) vorgelegt und unter Rühren auf 170 °C erwärmt. Nachdem das Reaktionsgemisch keine Epoxidgruppen mehr enthielt (ca. 4 Stunden) wurde im Vakuum bis etwa 190 °C destilliert. Es wurde eine dunkelgelbe Flüssigkeit mit einer OHZ von 84,6, einer VZ von 239 und einer SZ von 2,4 erhalten.

423 g des Umsetzungsproduktes von Sojaölepoxid mit Carbonsäuren wurden mit 6,9 g einer 30 gew.-%igen Lösung von Kaliumhydroxid in Methanol versetzt und analog Beispiel 1 bei 140 °C mit 660 g Ethylenoxid umgesetzt. Nach Entfernen der Ethylenoxidspuren im Vakuum und Neutralisation mit Milchsäure wurde eine dunkelgelbe Flüssigkeit mit einer OHZ von 54,7 erhalten.

### 5. Herstellung von ethoxyliertem OH-gruppenhaltigem Sojaöl aus mit Laurylalkohol umgesetztem Sojaölepoxid

In einem Rührkessel wurden 474 g Sojaölepoxid (Kenndaten wie in Beispiel 1 angegeben) und 745 g Laurylalkohol mit 3,6 g konzentrierter Schwefelsäure versetzt und auf 100 °C erwärmt. Nachdem das Reaktionsgemisch keine Epoxidgruppen mehr enthielt (ca. 3,5 Stunden) wurde mit 3,6 g Diethylethanolamin neutralisiert und der überschüssige Laurylalkohol im Vakuum bei 10 Pa und 135 °C abdestilliert. Es wurden 723 g Umsetzungsprodukt mit einer OHZ von 112, einer JZ von 20, einer VZ von 116 und einer SZ von 72 erhalten.

390 g des Umsetzungsproduktes von Sojaölepoxid mit Laurylalkohol wurden mit 4,0 g einer 30 gew.-%igen Lösung von Kaliumhydroxid in Methanol versetzt und analog Beispiel 1 bei 170 °C mit 610 g Ethylenoxid umgesetzt. Nach Entfernen der Ethylenoxidspuren im Vakuum und Neutralisation mit Milchsäure wurde eine gelbe Paste erhalten.

## Patentansprüche

1. Alkoxylierungsprodukte, erhältlich durch Umsetzung von Ethylenoxid, Propylenoxid und/oder Butylenoxid mit C₁₀₋₂₂-Carbonsäuremono-, -di- und/oder -triglyceriden natürlichen Ursprungs, die in Stellung 9, 10 und/oder 13, 14 Struktureinheiten der allgemeinen Formel 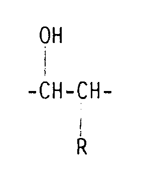 enthalten, in der
R ein Wasserstoffatom, eine OH-Gruppe oder eine OR¹-Gruppe,
R¹ eine Alkylgruppe mit 9 bis 18 C-Atomen, eine Alkenylgruppe mit 9 bis 18 C-Atomen oder eine 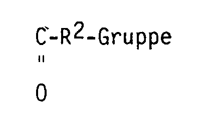
und
R² ein Wasserstoffatom, eine Alkylgruppe mit 1 bis 21 C-Atomen oder eine Alkenylgruppe mit 2 bis 21 C-Atomen
bedeuten.

2. Alkoxylierungsprodukte nach Anspruch 1, dadurch gekennzeichnet, daß der Alkylenoxidgehalt, bezogen auf nichtalkoxylierte Mono-, Di- und/oder Triglyceride, zwischen 2 und 400 Gew.-%, vorzugsweise zwischen 40 und 200 Gew.-%, liegt.

3. Alkoxylierungsprodukte nach einem oder beiden der Ansprüche 1 bis 2, dadurch gekennzeichnet, daß diese durch Umsetzung von Ethylenoxid und/oder Propylenoxid erhältlich sind.

4. Alkoxylierungsprodukte nach einem oder mehreren der Ansprüche 1 bis 3, dadurch gekennzeichnet, daß die Carbonsäurereste der Mono-, Di- und/oder Triglyceride 16 bis 22 C-Atome haben.

5. Verfahren zur Herstellung von Alkoxylierungsprodukten, dadurch gekennzeichnet, daß C₁₀₋₂₂-Carbonsäuremono-, -di- und/oder -triglyceride, die Carbonsäurereste enthalten, die in Stellung 9, 10 und/oder 13, 14 Struktureinheiten der allgemeinen Formel 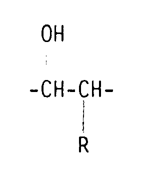 enthalten, in der
R ein Wasserstoffatom, eine OH-Gruppe oder eine OR¹-Gruppe,
R¹ eine Alkylgruppe mit 9 bis 18 C-Atomen, eine Alkenylgruppe mit 9 bis 18 C-Atomen oder eine 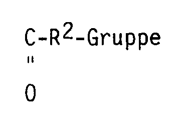 und
R² ein Wasserstoffatom, eine Alkylgruppe mit 1 bis 21 C-Atomen oder eine Alkenylgruppe mit 2 bis 21 C-Atomen
bedeuten, mit Ethylenoxid, Propylenoxid und/oder Butylenoxid bei Temperaturen zwischen 110 und 200 °C und Drücken zwischen 10⁵ und 2 · 10⁶ Pa umgesetzt werden.

6. Verfahren nach Anspruch 5, dadurch gekennzeichnet, daß die Alkoxylierungen bei Temperaturen zwischen 140 und 175 °C und Drücken zwischen 3 · 10⁵ und 5 · 10⁵ Pa durchgeführt werden.

7. Verfahren nach einem oder beiden der Ansprüche 5 oder 6, dadurch gekennzeichnet, daß die Alkoxylierungen in Gegenwart von Katalysatoren durchgeführt werden.

8. Verfahren nach einem oder mehreren der Ansprüche 5 bis 7, dadurch gekennzeichnet, daß Alkylenoxide in solchen Mengen eingesetzt werden, daß der Alkylenoxidgehalt der Alkoxylierungsprodukte, bezogen auf nicht alkoxylierte Mono-, Di- und/oder Triglyceride, zwischen 2 und 400 Gew.-%, vorzugsweise zwischen 40 und 200 Gew.-%, liegt.

9. Verfahren nach einem oder mehreren der Ansprüche 5 bis 8, dadurch gekennzeichnet, daß die Alkoxylierungen mit Ethylenoxid und/oder Propylenoxid durchgeführt werden.

10. Verfahren nach einem oder mehreren der Ansprüche 5 bis 9, dadurch gekennzeichnet, daß Mono-, Di- und/oder Triglyceride mit 16 bis 22 Kohlenstoffatomen in den Carbonsäureresten eingesetzt werden.

## Claims

1. Alkoxylation products obtainable by reaction of ethylene oxide, propylene oxide and/or butylene oxide with C₁₀₋₂₂ carboxylic acid mono-, di- and/or triglycerides of natural origin which, in the 9, 10 and/or 13, 14 position, contain structural units corresponding to the following general formula: 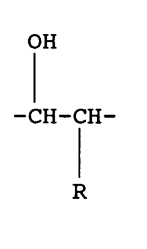 in which
R is a hydrogen atom, an OH group or an OR¹ group,
R¹ is a C₉₋₁₈ alkyl group, a C₉₋₁₈ alkenyl group or a 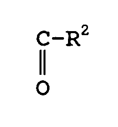 group and
R² is a hydrogen atom, a C₁₋₂₁ alkyl group or a C₂₋₂₁ alkenyl group.

2. Alkoxylation products as claimed in claim 1, characterized in that the alkylene oxide content, based on non-alkoxylated mono-, di and/or triglycerides, is between 2 and 400% by weight and preferably between 40 and 200% by weight.

3. Alkoxylation products as claimed in one or both of claims 1 and 2, characterized in that they are obtainable by reaction of ethylene oxide and/or propylene oxide.

4. Alkoxylation products as claimed in one or more of claims 1 to 3, characterized in that the carboxylic acids of the mono-, di- and/or triglycerides contain 16 to 22 carbon atoms.

5. A process for the production of alkoxylation products, characterized in that C₁₀₋₂₂ carboxylic acid mono-, di- and/or triglycerides which, in the 9, 10 and/or 13, 14 position, contain structural units corresponding to the following general formula: 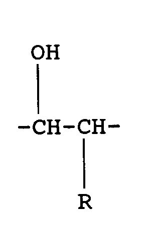 in which
R is a hydrogen atom, an OH group or an OR¹ group,
R¹ is a C₉₋₁₈ alkyl group, a C₉₋₁₈ alkenyl group or a 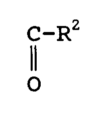 group and
R² is a hydrogen atom, a C₁₋₂₁ alkyl group or a C₂₋₂₁ alkenyl group,
are reacted with ethylene oxide, propylene oxide and/or butylene oxide at temperatures of 110 to 200°C and under pressures of 10⁵ to 2 · 10⁶ Pa.

6. A process as claimed in claim 5, characterized in that the alkoxylations are carried out at temperatures of 140 to 175°C under pressures of 3 · 10⁵ to 5 · 10⁵ Pa.

7. A process as claimed in one or both of claims 5 or 6, characterized in that the alkoxylations are carried out in the presence of catalysts.

8. A process as claimed in one or more of claims 5 to 7, characterized in that alkylene oxides are used in such quantities that the alkylene oxide content of the alkoxylation products, based on non-alkoxylated mono-, di- and/or triglycerides, is between 2 and 400% by weight and preferably between 40 and 200% by weight.

9. A process as claimed in one or more of claims 5 to 8, characterized in that the alkoxylations are carried out with ethylene oxide and/or propylene oxide.

10. A process as claimed in one or more of claims 5 to 9, characterized in that mono-, di- and/or triglycerides containing 16 to 22 carbon atoms in the carboxylic acids are used.

## Revendications

1. Produits d'alcoxylation accessibles par réaction de l'oxyde d'éthylène, l'oxyde de propylène et/ou l'oxyde de butylène avec des mono-, di- et/ou triglycérides d'acides carboxyliques en C₁₀ à C₂₂ d'origine naturelle qui renferment en position 9, 10 et/ou 13, 14 des éléments de structure de formule générale : 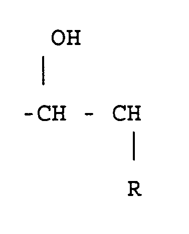 dans laquelle
R signifie un atome d'hydrogène, un groupe OH ou un groupe OR¹
R¹ signifie un groupe alcoyle ayant de 9 à 18 atomes de carbone, un groupe alcényle ayant de 9 à 18 atomes de carbone ou un groupe : 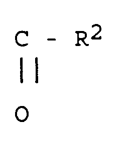 et R² signifie un atome d'hydrogène, un groupe alcoyle ayant de 1 à 21 atomes de carbone, ou un groupe alcényle ayant de 2 à 21 atomes de carbone.

2. Produits d'alcoxylation selon la revendication 1, caractérisés en ce que la teneur en oxyde d'alcoylène, rapportée aux mono-, di-, et/ou triglycérides non alcoxylés, se situe entre 2 et 400 % en poids, de préférence entre 40 et 200 % en poids.

3. Produits d'alcoxylation, selon l'une ou selon les deux revendications 1 à 2, caractérisés en ce qu'ils sont accessibles par réaction de l'oxyde d'éthylène et/ou de l'oxyde de propylène.

4. Produits d'alcoxylation selon l'une ou plusieurs des revendications 1 à 3, caractérisés en ce que les restes d'acide carboxylique des mono-, di- et/ou triglycérides, ont de 16 à 22 atomes de carbone.

5. Procédé d'obtention de produits d'alcoxylation caratérisé en ce que les mono-, di- et/ou triglycérides d'acide carboxylique en C₁₀ à C₂₂, qui renferment des restes d'acide carboxylique qui contiennent en position 9, 10 et/ou 13, 14 des éléments de structure de formule générale : 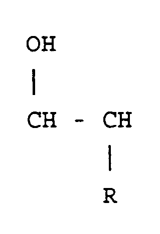 dans laquelle
R signifie un atome d'hydrogène, un groupe OH ou un groupe OR¹
R¹ signifie un groupe alcoyle ayant de 9 à 18 atomes de carbone ou un groupe alcényle ayant de 2 à 18 atomes de carbone ou un groupe : 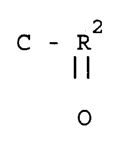 et R² signifie un atome d'hydrogène, un groupre alcocoyle ayant de 1 à 21 atomes de carbone ou un groupe alcényle ayant de 2 à 21 atomes de carbone sont mis à réagir avec l'oxyde d'éthylène, de l'oxyde de propylène et/ou de l'oxyde de butylène à des températures comprises entre 110 et 200° et à des pressions comprises entre 10⁵ et 2.10⁶ Pa.

6. Procédé selon la revendication 5, caractérisé en ce que les alcoxylations sont effectuées à des températures comprises entre 140 et 175°C et à des pressions comprises entre 3.10⁵ et 5.10⁵ Pa.

7. Procédé selon l'une ou les deux revendications 5 et 6, caractérisé en ce que l'on effectue les alcoxylations en présence de catalyseurs.

8. Procédé selon l'une ou plusieurs des revendications 5 à 7 caractérisé en ce que l'on met en oeuvre les oxydes d'alcoylène en quantités telles que la teneur en oxyde d'alcoylène des produits d'alcoxylation rapporté aux mono-, di- triglycérides non alcoxylés, se situe entre 2 et 400 % en poids, de préférence entre 40 et 200 % en poids.

9. Procédé selon l'une ou plusieurs des revendications 5 à 8, caractérisé en ce que l'on effectue les alcoxylations avec de l'oxyde d'éthylène et/ou de l'oxyde de propylène.

10. Procédé selon l'une ou plusieurs des revendications 5 à 9, caractérisé en ce que sont mis en oeuvre des mono-, di- et/ou triglycérides ayant de 16 à 22 atomes de carbone dans les restes d'acide carboxylique,.
